# EUROPEAN PATENT SPECIFICATION

(11) **EP 3 427 698 B1**
(45) Date of publication and mention of the grant of the patent: **08.01.2020**
(21) Application number: 18180690.2
(22) Date of filing: 29.06.2018
(51) Int. Cl.: A61F 2/38

(54) **SYSTEM FOR AN ORTHOPAEDIC JOINT REPLACEMENT PROCEDURE**
SYSTEM FÜR EIN ORTHOPÄDISCHES GELENKERSATZVERFAHREN
SYSTÈME POUR PROCÉDURE DE REMPLACEMENT D'ARTICULATION ORTHOPÉDIQUE

(30) Priority: 14.07.2017 US 201715650353
(43) Date of publication of application: 16.01.2019
(73) Proprietor: DePuy Ireland Unlimited Company, County Cork (IE)
(72) Inventor: SCHMIT, Matthew D., Warsaw, Indiana 46581 (US)
(74) Representative: Curran, Clair

(56) References cited:
- CN-U- 205 459 218
- FR-A1- 2 980 104
- FR-A1- 3 045 313
- US-A- 4 888 021

## Description

### TECHNICAL FIELD

The present disclosure relates generally to an orthopaedic prosthesis system, including prosthetic components for assembly during an orthopaedic joint replacement procedure, and, more particularly, to orthopaedic prosthetic components for assembly during a knee replacement procedure. The closest prior art is document FR 3045313 A1, which defines the preamble of claim 1.

### BACKGROUND

Movement (e.g., flexion and extension) of the natural human knee involves movement of the femur and the tibia. Specifically, during flexion and extension, the distal end of the femur and the proximal end of the tibia articulate relative to one another through a series of complex movements. Damage (e.g., trauma) or disease can deteriorate the bones, articular cartilage, and ligaments of the knee, which can ultimately affect the ability of the natural knee to function in such a manner. As a result, knee prostheses have been developed and implanted into surgically prepared ends of the femur and tibia.

A typical knee prosthesis for a total knee replacement, for example, includes a tibial component or tibial tray coupled to the patient's tibia, a femoral component coupled to the patient's femur, and a bearing component positioned between the tibial tray and the femoral component and including a bearing surface to accommodate the condyles of the femoral component. A constrained knee prosthesis, however, may be used when a patient's collateral ligaments have been damaged or are otherwise not able to provide adequate support and stability to the knee. One such constrained knee prosthesis is a hinged knee prosthesis, which typically includes a hinge mechanism to couple the femoral component to one or both of the bearing component and the tibial components in order to constrain and mechanically link the components of the knee prosthesis together.

### SUMMARY

The invention is defined in claim 1 and concerns an orthopaedic prosthetic assembly including a femoral component configured to be attached to a distal end of a patient's femur. The femoral component includes a pair of condyles. An intercondylar notch is defined between the condyles. A posterior bore extends in a medial-lateral direction through the pair of condyles. A tibial tray is configured to be attached to a proximal end of a patient's tibia. The tibial tray includes a platform and a post extending distally from the platform. A tibial insert includes a body having a pair of surfaces configured to engage corresponding condyle surfaces of the condyles of the femoral component. A stem extends distally from the body and sized to be received in a cavity defined in the tibial tray. A spine extends proximally from the body. A pin hole extends through the spine of the tibial insert. A pair of bushings is sized and shaped to be positioned in the pin hole of the spine. A hinge pin is sized and shaped to be positioned in the pair of bushings and the posterior bore to couple the tibial insert to the femoral component. The spine includes a medial opening, a lateral opening, and an inner wall extending between the medial and the lateral openings to define the pin hole. Each bushing has an annular flange. An elongated body extends from the annular flange. The elongated body includes a retaining tab. The retaining tab of a first bushing is sized and shaped to secure to the retaining tab of a second bushing to secure the pair of bushings to the spine.

In some embodiments, the retaining tab of each bushing may have a pair of angled surfaces extending from the outer surface of the elongated body to an end surface. In some embodiments, the retaining tab of each bushing may have a longitudinal axis and the angled surfaces may angle outward from the longitudinal axis from elongated body to the end surface. In some embodiments, an angled surface of the retaining tab of the first bushing may engage an angled surface of the retaining tab of the second bushing to secure the first bushing to the second bushing.

In some embodiments, the retaining tab may have a plurality of retaining tabs and the elongated body may have a plurality of slots. Each slot of the plurality of slots may be positioned between adjacent retaining tabs of the plurality of retaining tabs and each retaining tab of the plurality of retaining tabs may be positioned between adjacent slots of the plurality of slots. In some embodiments, each of the plurality of retaining tabs of the first bushing may be positioned within one of the plurality of slots of the second bushing to secure the first bushing to the second bushing. In some embodiments, each of the plurality of retaining tabs of the second bushing may be positioned within one of the plurality of slots of the first bushing to secure the first bushing to the second bushing.

The invention may be used in a method of implanting an orthopaedic prosthetic assembly that includes attaching a femoral component to a distal end of a patient's femur. The femoral component includes a pair of condyles. An intercondylar notch is defined between the condyles. A posterior bore extends in a medial-lateral direction through the pair of condyles. The method also includes attaching a tibial tray to a proximal end of a patient's tibia. The tibial tray includes a platform and a post extending distally from the platform. The method also includes inserting a stem of a tibial insert in a cavity defined in the tibial tray. The tibial insert includes a body having a pair of surfaces configured to engage corresponding condyle surfaces of the condyles of the femoral component. The stem extends distally from the body. A spine extends proximally from the body. The spine includes a medial opening, a lateral opening, and an inner wall extending between the medial and the lateral openings to define the pin hole. The method also includes inserting a pair of bushings in the pin hole of the spine such that a retaining tab of each bushing secures the bushings within the pin hole. The method also includes inserting a hinge pin in the pair of bushings and the posterior bore to couple the tibial insert to the femoral component.

In some constructions, the inner wall of the spine may have a first diameter, and the retaining tab may have an outer wall having a second diameter. The second diameter may be greater than the first diameter. The method may also require inserting the pair of bushings in the pin hole such that the outer wall of the retaining tab compresses to decrease the second diameter to be equal to the first diameter to secure the bushing to the inner wall of the spine.

In some constructions, the retaining tab may be a plurality of retaining tabs and the elongated body may have a plurality of slots. Each slot of the plurality of slots may be positioned between adjacent retaining tabs of the plurality of retaining tabs, and each retaining tab of the plurality of retaining tabs may be positioned between adjacent slots of the plurality of slots. The method may also require inserting the pair of bushings in the pin hole such that each of the plurality of retaining tabs of a first bushing is positioned within one of the plurality of slots of a second bushing, and each of the plurality of retaining tabs of the second bushing is positioned within one of the plurality of slots of the first bushing to secure the first bushing to the second bushing.

### BRIEF DESCRIPTION OF THE DRAWINGS

The detailed description particularly refers to the following figures, in which:
FIG. 1 is a perspective view of an orthopaedic prosthetic assembly having a femoral component that is hinged to a tibial component;
FIG. 2 is an exploded view of the orthopaedic prosthetic assembly of FIG. 1;
FIG. 3 is a perspective view of a bushing formed in accordance with an embodiment;
FIG. 4 is a cross-sectional view taken along the line 4-4 in FIG. 3;
FIG. 5 is an elevation view of the bushing of FIG. 3;
FIG. 6 is a cross-sectional view of the tibial insert shown in FIG. 1 having a pair of the bushings shown in FIG. 3 assembled therein;
FIG. 7 is a perspective view of an alternative bushing for use in the orthopaedic prosthetic assembly of FIG. 1;
FIG. 8 is a cross-sectional view taken along the line 8-8 of FIG. 7;
FIG. 9 is a cross-sectional view of the tibial insert similar to Fig. 6 showing a pair of the bushings shown in FIG. 7 assembled therein;
FIG. 10 is an elevation view of another alternative bushing for use in the orthopaedic prosthetic assembly of FIG. 1;
FIG. 11 is a detail view of the bushing of FIG. 10;
FIG. 12 is a plan view of the bushing of FIG. 10;
FIG. 13 is a cross-sectional view taken along the line 13-13 of FIG. 10; and
FIG. 14 is a cross-sectional view of the tibial insert similar to Fig. 6 showing a pair of the bushings shown in FIG. 10 assembled therein.

### DETAILED DESCRIPTION OF THE DRAWINGS

While the concepts of the present disclosure are susceptible to various modifications and alternative forms, specific exemplary embodiments thereof have been shown by way of example in the drawings and will herein be described in detail. It should be understood, however, that there is no intent to limit the concepts of the present disclosure to the particular forms disclosed, but on the contrary, the intention is to cover all modifications falling within the scope of the invention as defined by the appended claims.

Terms representing anatomical references, such as anterior, posterior, medial, lateral, superior, inferior, etcetera, may be used throughout the specification in reference to the orthopaedic implants and orthopaedic surgical instruments described herein as well as in reference to the patient's natural anatomy. Such terms have well-understood meanings in both the study of anatomy and the field of orthopaedics. Use of such anatomical reference terms in the written description and claims is intended to be consistent with their well-understood meanings unless noted otherwise.

The exemplary embodiments of the present disclosure are described and illustrated below to encompass prosthetic knee joints and knee joint components, as well as methods of implanting and reconstructing knee joints. It will also be apparent to those of ordinary skill in the art that the preferred embodiments discussed below are exemplary in nature and may be reconfigured without departing from the scope of the present invention. However, for clarity and precision, the exemplary embodiments as discussed below may include optional features that one of ordinary skill should recognize as not being a requisite to fall within the scope of the present invention.

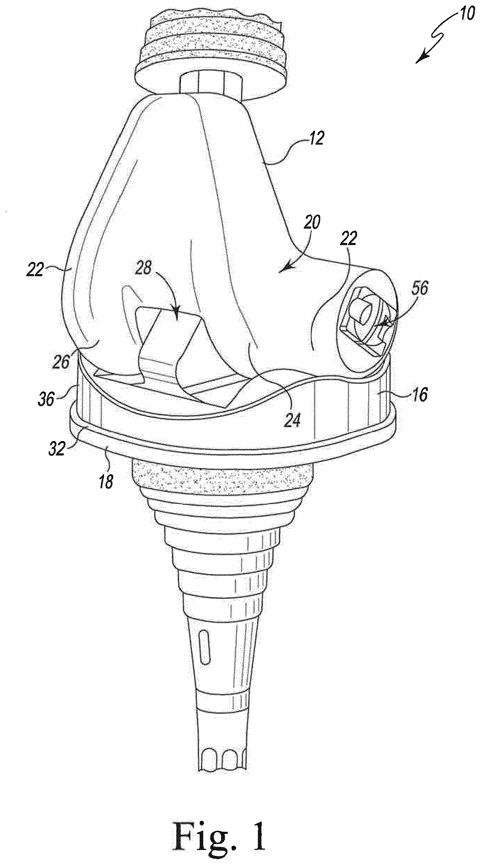

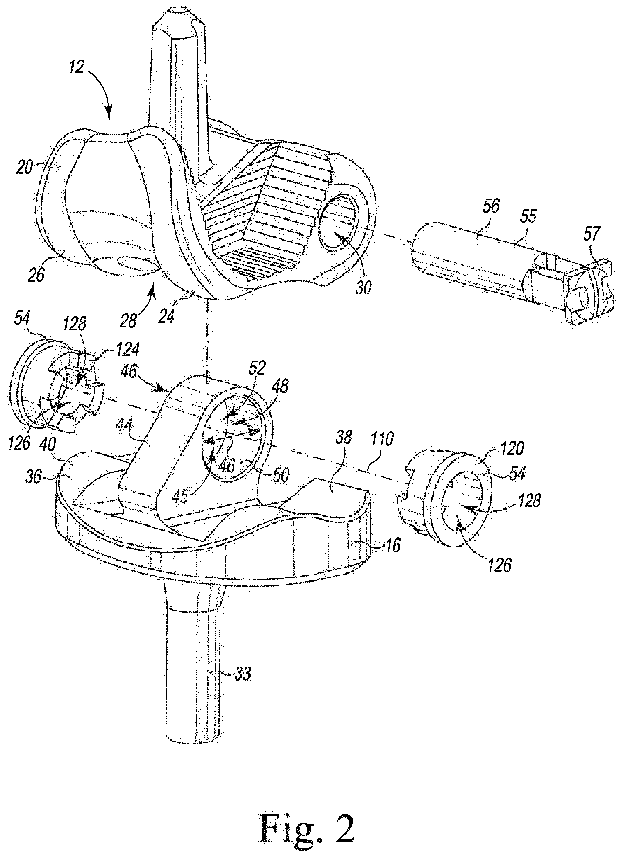

Referring now to FIGS. 1 and 2, an orthopaedic knee prosthesis 10 is shown. The orthopaedic knee prosthesis 10 includes a femoral component 12 and a tibial insert 16 that is coupled to the femoral component 12 via a hinge mechanism. The femoral component 12 is configured to be implanted into a prepared end of the patient's femur (not shown) and includes a body 20 having a pair of spaced-apart lateral and medial condyles 22. The condyles 22 includes respective lateral and medial condyle surfaces 24, 26, which are curved and shaped to correspond to surfaces of the tibial insert 16. An intercondylar notch 28 is defined between the lateral and medial condyles 22. The femoral component 12 also includes a posterior bore 30 that extends in a medial-lateral direction through lateral and medial condyles 22. As described in greater detail below, the bore 30 forms part of the hinge mechanism 14 and is sized to receive a hinge pin 56.

The prosthesis 10 also includes a tibial component or tray 18 that is configured to be implanted into a prepared end of the patient's tibia (not shown). The tibial tray 18 includes a platform 32 having a generally flat top surface with a cavity (not shown) extending therethrough. A tibial stem extends from the platform 32.

The tibial insert 16 includes a body 36 having a generally flat bottom surface configured to rest upon the generally flat top surface of the platform 32. The tibial insert 16 further includes a lateral bearing surface 38 and a medial bearing surface 40. The bearing surfaces 38, 40 are configured to articulate with a lateral condyle surface 24 and a medial condyle surface 26, respectively, of the femoral component 12. A stem 33 extends distally from the body 36 and is sized to be received in the cavity defined in the tibial tray 18. A spine 44 extends proximally from the body 36. The spine 44 includes a medial opening 46, a lateral opening 48, and an inner wall 50 extending between the medial opening 46 and the lateral opening 48. The inner wall 50 defines a cylindrical pin hole 52 through the spine 44. The cylindrical pin hole 42 has a diameter 43 and defines a passageway 45 through spine 44. The passageway 45 extends between the medial opening 46 and the lateral opening 48.

A pair of bushings 54 are sized and shaped to be positioned in the pin hole 52 of the spine 44. A first end 120 of the bushing 54 has an opening 122 formed therein. An opening 126 is formed through a second end 124 of the bushing 54. A passageway 128 extends through the bushing 54 between the opening 122 and the opening 126. The passageway 128 extends along a longitudinal axis 110 of the bushing 54. A hinge pin 56 is sized and shaped to be positioned in the pair of bushings 54 and the posterior bore 30 of the femoral component 12 to couple the tibial insert 16 to the femoral component 12. The hinge pin 56 has an elongated body 55 extending from a head end 57. The elongated body 55 extends through the passageway 128 of each bushing 54 and the posterior bore 30. The hinge pin 56 is locked into position within the bushings 54 and the posterior bore 30.

The components of the knee prosthesis 10 that engage the natural bone, such as the femoral component 12 and the tibial tray 18 may be constructed from a biocompatible metal, such as titanium or cobalt chrome alloy for example. The bone engaging surfaces of these components may be textured to facilitate cementing the component to the bone. Such surfaces may also be porous coated to promote bone ingrowth for permanent fixation. Alternatively, the tibial insert 16 may be constructed from a material that allows for smooth articulation and rotation between the tibial insert 16 and the adjacent femoral and tibial components 12, 18. One such material is ultra-high molecular weight polyethylene (UHMWPE). Of course, the tibial insert 16 may be made from other suitable polymers as well.

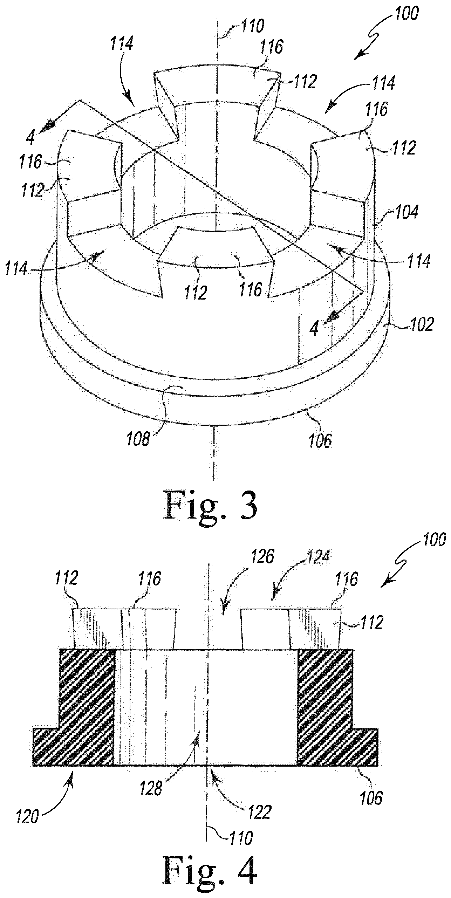

Referring to FIG. 3, an embodiment of the bushing 54 includes an annular flange 102 and an elongated body 104 extending from the annular flange 102. The bushing 54 may be constructed from a biocompatible metal, such as titanium or cobalt chrome alloy for example. Alternatively, the bushing 54 may be constructed from a polymer, for example ultra-high molecular weight polyethylene (UHMWPE). The annular flange 102 includes a lateral surface 106 and an opposite medial surface 108. The lateral surface 106 of the annular flange 102 forms the first end 120 of the bushing 54. The elongated body 104 extends from the medial surface 108 along a longitudinal axis 110. A plurality of retaining tabs 112 extend longitudinally from the elongated body 104. A plurality of slots 114 are formed between the retaining tabs 112. Each slot 114 is positioned between adjacent retaining tabs 112. Each retaining tab 112 is positioned between adjacent slots 114. In the illustrative embodiment, the bushing 54 includes four retaining tabs 112 and four slots 114. In some embodiments, the bushing 54 may include any number of retaining tabs 112 and slots 114. For example, the bushing 54 may include at least two retaining tabs 112 and at least two slots 114. Each retaining tab 112 is defined by an end surface 116 and a pair of side surfaces 118 (described in more detail below). The end surfaces 116 of the retaining tabs 112 form the second end 124 of the bushing 54.

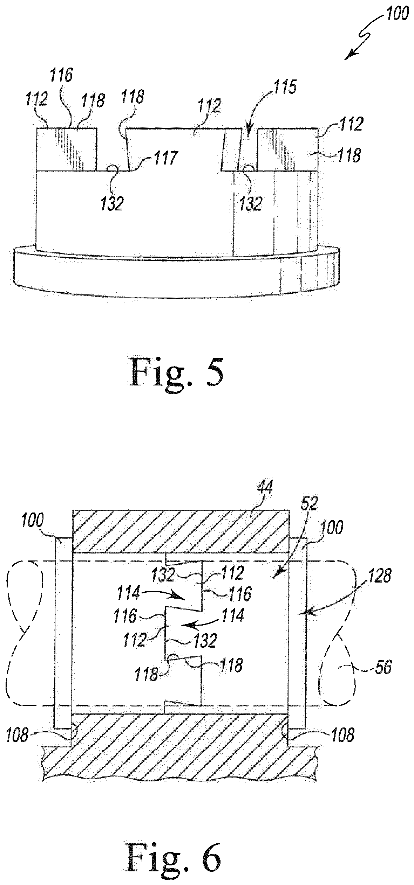

Referring to FIG. 5, each retaining tab 112 extends from a base 117 to the end surface 116. When viewed in a side elevation, as illustrated in Fig.5, the end surface 116 is wider than the base 117. That is, each side surface 118 extends outward from the base 117 to the end surface 116. Each slot 114 is defined by the side surfaces 118 of adjacent retaining tabs 112 and a bottom surface 132. Each slot 114 includes an opening 115 that is smaller than a width of the bottom surface 132. The side surfaces 118 angle from the bottom surface 132 to the opening 115. That is, each side surface 118 extends inward from the bottom surface 132 to the opening 115.

Each retaining tab 112 is formed to be approximately the same shape and size. Likewise, each slot 114 is formed to be approximately the same shape and size. The slots 114 are sized and shaped as mirror images of the size and shape of the retaining tabs 112. The end surface 116 of each retaining tab 112 has a surface area that is substantially equal to a surface area of the bottom surface 132 of each slot 114. The end surface 116 of each retaining tab 112 is wider than a base 117 of each retaining tab 112. The side surfaces 118 angle from the base 117 to the end surface 116. When a first bushing 54 is coupled to a second bushing 54, the retaining tabs 112 deform to allow the wider end surface 116 of each retaining tab 112 to be inserted through the opening 115 of the respective slot 114. The side surfaces 118 of the first bushing 54 engage the side surfaces 118 of the second bushing 54 in a substantially flush configuration when the first bushing 54 and the second bushing 54 are coupled.

Referring to FIG. 6, a user advances a pair of bushings 54 into the pin hole 52 of the spine 44. The bushings 54 are positioned within the pin hole 52 such that the medial surface 108 of the annular flange 102 is positioned flush with the spine 44. A first bushing 54 engages a second bushing 54 within the pin hole 52. Specifically, each retaining tab 112 of the first bushing 54 is positioned within a slot 114 of the second bushing 54. The end surface 116 of each retaining tab 112 of the first bushing 54 is positioned flush with the bottom surface 132 of the respective slot 114 of the second bushing 54. Likewise, each retaining tab 112 of the second bushing 54 is positioned within a slot 114 of the first bushing 54. The end surface 116 of each retaining tab 112 of the second bushing 54 is positioned flush with the bottom surface 132 of the respective slot 114 of the first bushing 54. The side surface 118 of each retaining tab 112 of the first bushing 54 engages the side surface 118 of a corresponding retaining tab 112 of the second bushing 54. Accordingly, the retaining tabs 112 of the first bushing 54 engage the retaining tabs 112 of the second bushing 54 to lock the first bushing 54 to the second bushing 54.

With the bushings 54 secured within the pin hole 52, the user positions the femoral component 12 on the spine 44 such that the posterior bore 30 of the femoral component 12 is aligned with the passageways 128 of the bushings 54. The user then advances the hinge pin 56 through the passageways 128 of the bushings 54 and the posterior bore 30 of the femoral component 12 to couple the tibial insert 16 to the femoral component 12.

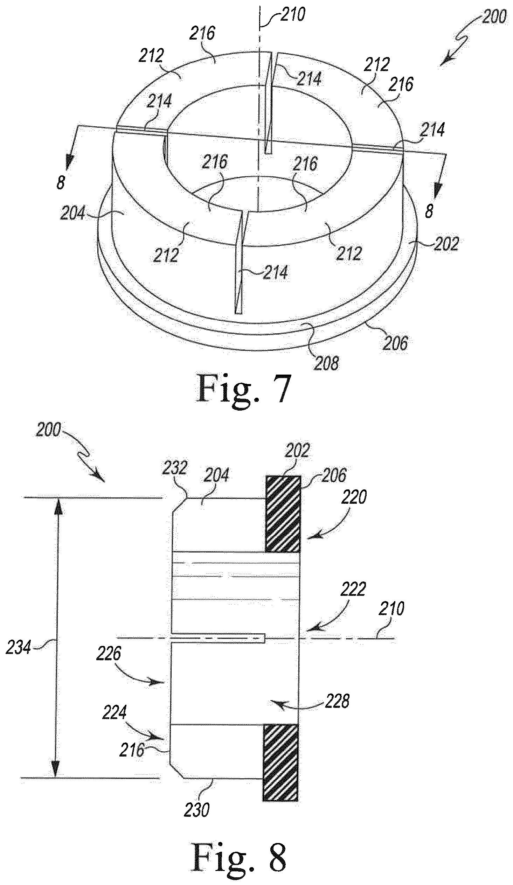

Referring to FIG. 7, a bushing 200, which does not form part of the present invention, includes an annular flange 202 and an elongated body 204 extending from the annular flange 202. The bushing 200 may be constructed from a biocompatible metal, such as titanium or cobalt chrome alloy for example. Alternatively, the bushing 200 may be constructed from a polymer, for example ultra-high molecular weight polyethylene (UHMWPE). The annular flange 202 includes a lateral surface 206 and an opposite medial surface 208. The elongated body 204 extends from the medial surface 208 along a longitudinal axis 210. A plurality of retaining tabs 212 extend longitudinally from the elongated body 204 to a respective end surface 216. A plurality of slots 214 are formed between the retaining tabs 212. Each slot 214 is positioned between adjacent retaining tabs 212. Each retaining tab 212 is positioned between adjacent slots 214. The slots 214 form a gap between each retaining tab 212. In the illustrative example, the bushing 200 includes four retaining tabs 212 and four slots 214. In some embodiments, the bushing 200 may include any number of retaining tabs 212 and slots 214. For example, the bushing 200 may include at least two retaining tabs 212 and at least two slots 214. The retaining tabs 212 are deformable such that the retaining tabs 212 can collapse together. When the retaining tabs 212 collapse, the retaining tabs 212 are moved into contact with one another into the gaps defined by the slots 214

As illustrated in FIG. 8, the lateral surface 206 of the annular flange 202 forms a first end 220 of the bushing 200. The lateral surface 206 includes an opening 222 formed therein. The end surfaces 216 of the retaining tabs 212 form a second end 224 of the bushing 200. An opening 226 is formed through the end surfaces 216. A passageway 228 extends through the bushing 200 between the opening 222 and the opening 226. The passageway 228 extends along the longitudinal axis 210 of the elongated body 204.

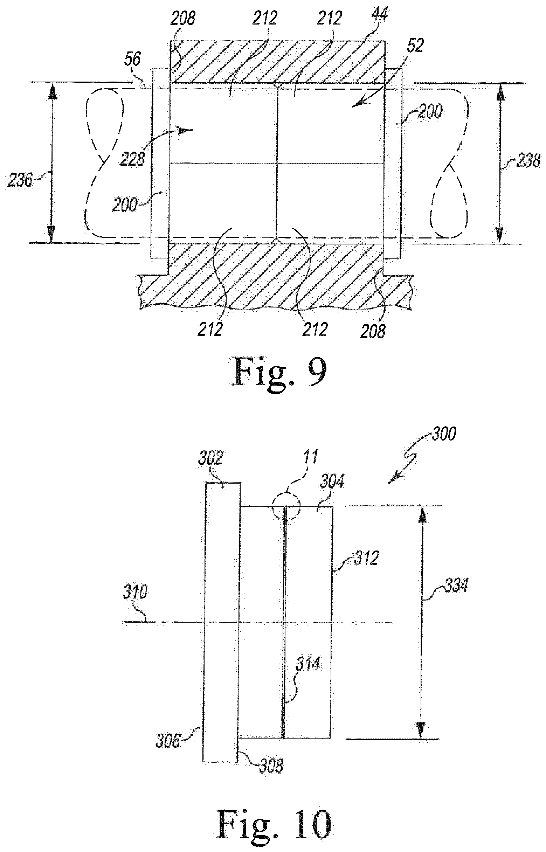

The elongated body 204 includes an outer surface 230. The outer surface 230 forms an outer surface 232 of the retaining tabs 212. When the bushing 200 is not assembled within the pin hole 52 of the spine 44, the outer surface 230 has an unassembled diameter 234. The diameter 234 is the diameter of the elongated body 204. When the retaining tabs 212 are collapsed together, the outer surface 230 has an assembled diameter 238 (shown in FIG. 9) that is less than the unassembled diameter 234.

Referring to FIG. 9, the pin hole 52 of the spine 44 has a diameter 236. The diameter 236 of the pin hole 52 is less than the unassembled diameter 234 of the bushing 200. As illustrated in FIG. 9, during assembly, a user advances a pair of bushings 200 into the pin hole 52 of the spine 44. The bushings 200 are positioned within the pin hole 52 such that the medial surface 208 of the annular flange 202 is positioned flush with the spine 44. The bushings 200 are advanced into the pin hole 52 such that the retaining tabs 212 of each bushing 200 compress as the bushing 200 is advanced. That is, the retaining tabs 212 are compressed together into the gap created by the slots 214. When the bushings 200 are positioned within the pin hole 52, as illustrated in FIG. 9, the retaining tabs 212 are compressed such that the outer surface 230 of the bushing 200 has the assembled diameter 238 that is equal to the diameter 236 of the pin hole 52. In this configuration, the outer surface 230 of the bushing 200 engages the inner wall 50 of the pin hole 52 to secure the bushings 200 to the spine 44.

With the bushings 200 secured within the pin hole 52, the user positions the femoral component 12 on the spine 44 such that the posterior bore 30 of the femoral component 12 is aligned with the passageways 228 of the bushings 200. The user then advances the hinge pin 56 through the pair of bushings 200 and the posterior bore 30 of the femoral component 12 to couple the tibial insert 16 to the femoral component 12.

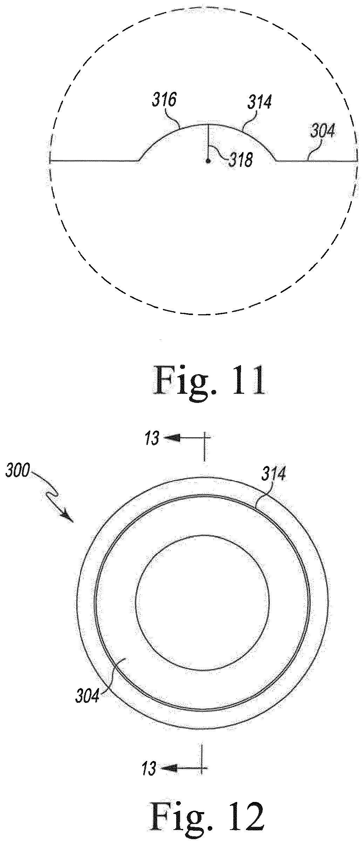

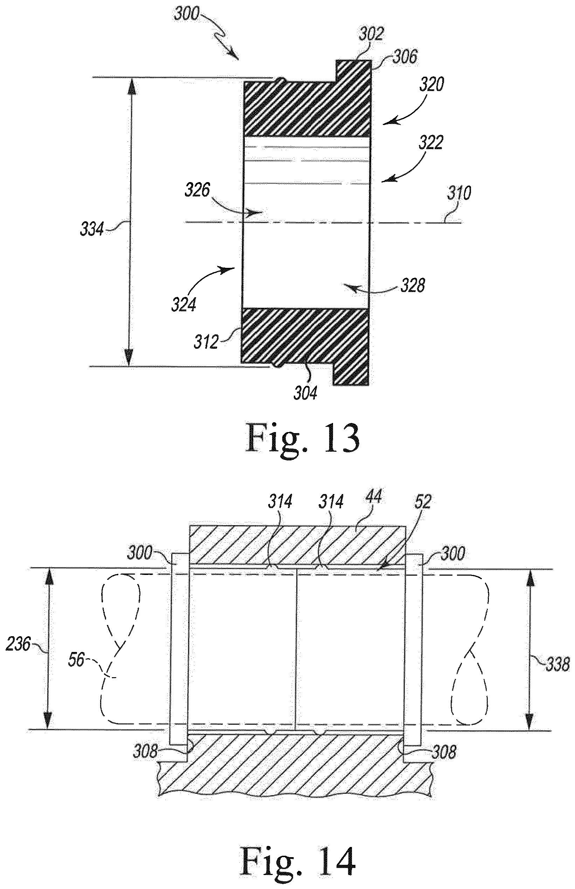

Referring to FIG. 10, an alternative bushing 300, which does not form part of the present invention, includes an annular flange 302 and an elongated body 304 extending from the annular flange 302. The bushing 300 may be constructed from a biocompatible metal, such as titanium or cobalt chrome alloy for example. Alternatively, the bushing 300 may be constructed from a polymer, for example ultra-high molecular weight polyethylene (UHMWPE). The annular flange 302 includes a lateral surface 306 and an opposite medial surface 308. The elongated body 304 extends from the medial surface 308 along a longitudinal axis 310 to an end surface 312. A retaining tab 314 extends circumferentially around the elongated body 304, as shown in FIG. 12. The retaining tab 314 is an annular rim having an outer curved wall 316. As shown in FIG. 11, the outer curved wall 316 has a radius of curvature 318. For example, the radius of curvature 318 may be approximately 0.96 mm. The retaining tab 314 is formed from a deformable material that enables the outer curved wall 316 to collapse under pressure. Referring back to FIG. 10, when the bushing 300 is not assembled within the pin hole 52 of the spine 44, the outer curved wall 316 has an unassembled diameter 334. The retaining tab 314 is deformable such that the retaining tab 314 may deform under pressure to alter the diameter 334 of the outer curved wall 316 to an assembled diameter 338 (shown in FIG. 14).

As illustrated in FIG. 13, the lateral surface 306 of the annular flange 302 forms a first end 320 of the bushing 300. The lateral surface 306 includes an opening 322 formed therein. The end surface 312 of the elongated body 304 form a second end 324 of the bushing 300. An opening 326 is formed through the end surface 312. A passageway 328 extends through the bushing 300 between the opening 322 and the opening 326. The passageway 328 extends along the longitudinal axis 310 of the elongated body 304.

Referring to FIG. 14, the pin hole 52 of the spine 44 has the diameter 236. The diameter 236 of the pin hole 52 is less than the unassembled diameter 334 of the retaining tab 314. As illustrated in FIG. 14, during assembly, a user advances a pair of bushings 300 into the pin hole 52 of the spine 44. The bushings 300 are positioned within the pin hole 52 such that the medial surface 308 of the annular flange 302 is positioned flush with the spine 44. The bushings 300 are advanced into the pin hole 52 such that the retaining tab 314 of each bushing 300 deforms as the bushing 300 is advanced. That is, the retaining tabs 314 are compressed, such that when the bushings 300 are positioned within the pin hole 52, as illustrated in FIG. 14, the retaining tabs 314 have the assembled diameter 338 that is equal to the diameter 236 of the pin hole 52. In this configuration, the outer curved wall 316 of each retaining tab 314 engages the inner wall 50 of the pin hole 52 to secure the bushings 300 to the spine 44.

With the bushings 300 secured within the pin hole 52, the user positions the femoral component 12 on the spine 44 such that the posterior bore 30 of the femoral component 12 is aligned with the passageways 328 of the bushings 300. The user then advances the hinge pin 56 through the pair of bushings 300 and the posterior bore 30 of the femoral component 12 to couple the tibial insert 16 to the femoral component 12.

While the disclosure has been illustrated and described in detail in the drawings and foregoing description, such an illustration and description is to be considered as exemplary and not restrictive in character, it being understood that only illustrative embodiments have been shown and described.

There are a plurality of advantages of the present disclosure arising from the various features of the devices and assemblies described herein. It will be noted that alternative embodiments of the devices and assemblies of the present disclosure may not include all of the features described yet still benefit from at least some of the advantages of such features. Those of ordinary skill in the art may readily devise their own implementations of the devices and assemblies that incorporate one or more of the features of the present invention and fall within the scope of the present invention as defined by the appended claims.

## Claims

1. An orthopaedic prosthetic assembly (10), comprising:
a femoral component (12) configured to be attached to a distal end of a patient's femur, the femoral component including (i) a pair of condyles (22), (ii) an intercondylar notch (28) defined between the condyles, and (iii) a posterior bore (30) extending in a medial-lateral direction through the pair of condyles,
a tibial tray (18) configured to be attached to a proximal end of a patient's tibia, the tibial tray including a platform (32) and a post (extending distally from the platform,
a tibial insert (16) including (i) a body (36) having a pair of surfaces (38, 40) configured to engage corresponding condyle surfaces (24, 26) of the condyles of the femoral component, (ii) a stem (33) extending distally from the body and sized to be received in a cavity defined in the tibial tray, (iii) a spine (44) extending proximally from the body, and (iv) a pin hole (42) extending through the spine of the tibial insert, and a hinge pin (56), wherein the spine includes a medial opening (46), a lateral opening (48), and an inner wall (50) extending between the medial and the lateral openings to define the pin hole, **characterised by**
a pair of bushings (54) sized and shaped to be positioned in the pin hole of the spine,
wherein the hinge pin (56) is sized and shaped to be positioned in the pair of bushings and the posterior bore to couple the tibial insert to the femoral component, and
wherein each bushing has an annular flange (102), and an elongated body (104) extending from the annular flange, the elongated body including a retaining tab (112), the retaining tab of a first bushing being sized and shaped to secure to the retaining tab of a second bushing to secure the pair of bushings to the spine.

2. The orthopaedic prosthetic assembly (10) of claim 1, wherein the retaining tab (112) of each bushing (54) includes a pair of angled surfaces (118) extending from the outer surface (116) of the elongated body (104) to an end surface (117).

3. The orthopaedic prosthetic assembly (10) of claim 2, wherein the retaining tab (112) of each bushing (54) includes a longitudinal axis (110) and the angled surfaces angle outward from the longitudinal axis of the elongated body (104) to the end surface (117).

4. The orthopaedic prosthetic assembly (10) of claim 3, wherein an angled surface (118) of the retaining tab (112) of the first bushing (54) engages an angled surface of the retaining tab of the second bushing to secure the first bushing to the second bushing.

5. The orthopaedic prosthetic assembly (10) of claim 4, wherein the retaining tab (112) includes a plurality of retaining tabs and the elongated body (104) includes a plurality of slots (114), wherein each slot of the plurality of slots is positioned between adjacent retaining tabs of the plurality of retaining tabs and each retaining tab of the plurality of retaining tabs is positioned between adjacent slots of the plurality of slots.

6. The orthopaedic prosthetic assembly (10) of claim 5, wherein each of the plurality of retaining tabs (112) of the first bushing (54) is positioned within one of the plurality of slots (114) of the second bushing to secure the first bushing to the second bushing.

7. The orthopaedic prosthetic assembly (10) of claim 6, wherein each of the plurality of retaining tabs (112) of the second bushing (54) is positioned within one of the plurality of slots (114) of the first bushing to secure the first bushing to the second bushing.

## Patentansprüche

1. Orthopädische Prothesenanordnung (10), umfassend:
eine Femurkomponente (12), die konfiguriert ist, an einem distalen Ende des Femurs eines Patienten angebracht zu werden, wobei die Femurkomponente (i) ein Paar Kondylen (22), (i) eine zwischen den Kondylen definierte interkondyläre Kerbe (28) und (iii) eine posteriore Bohrung (30) enthält, die sich in medial-lateraler Richtung durch das Kondylenpaar erstreckt,
eine Tibiaschale (18), die konfiguriert ist, an einem proximalen Ende der Tibia eines Patienten angebracht zu werden, wobei die Tibiaschale eine Plattform (32) und einen Pfosten (der sich distal von der Plattform erstreckt) enthält,
einen Tibiaeinsatz (16) mit (i) einem Körper (36) mit einem Paar Oberflächen (38, 40), die konfiguriert sind, mit entsprechenden Kondylenoberflächen (24, 26) der Kondylen der Femurkomponente in Eingriff zu kommen, (ii) einem Schaft (33) sich distal von dem Körper erstreckt und so bemessen ist, dass er in einem Hohlraum aufgenommen werden kann, der in der Tibiaschale definiert ist, (iii) einem Dorn (44), der sich proximal von dem Körper erstreckt, und (iv) einem Stiftloch (42), das sich durch den Dorn des Tibiaeinsatzes erstreckt, und
einen Scharnierstift (56),
wobei der Dorn eine mediale Öffnung (46), eine laterale Öffnung (48) und eine innere Wand (50) enthält, die sich zwischen der medialen und der lateralen Öffnung erstreckt, um das Stiftloch zu definieren, **gekennzeichnet durch** ein Paar Buchsen (54), die so bemessen und geformt sind, dass sie in dem Stiftloch des Dorn positioniert werden können,
wobei der Gelenkstift (56) so bemessen und geformt ist, dass er in dem Paar Buchsen und der posterioren Bohrung positioniert werden kann, um den Tibiaeinsatz mit der Femurkomponente zu koppeln, und
wobei jede Buchse einen ringförmigen Flansch (102) und einen länglichen Körper (104) aufweist, der sich von dem ringförmigen Flansch erstreckt, wobei der längliche Körper eine Haltelasche (112) enthält, wobei die Haltelasche einer ersten Buchse so bemessen und geformt ist, dass sie an der Haltelasche einer zweiten Buchse befestigt werden kann, um das Paar Buchsen an der Wirbelsäule zu befestigen.

2. Orthopädische Prothesenanordnung (10) nach Anspruch 1, wobei die Haltelasche (112) jeder Buchse (54) ein Paar abgewinkelter Flächen (118) enthält, die sich von der Außenfläche (116) des länglichen Körpers (104) zu einer Endfläche (117) erstrecken.

3. Orthopädische Prothesenanordnung (10) nach Anspruch 2, wobei die Haltelasche (112) jeder Buchse (54) eine Längsachse (110) enthält und die abgewinkelten Flächen von der Längsachse des länglichen Körpers (104) nach außen zu der Endfläche (117) abgewinkelt sind.

4. Orthopädische Prothesenanordnung (10) nach Anspruch 3, wobei eine abgewinkelte Fläche (118) der Haltelasche (112) der ersten Buchse (54) in eine abgewinkelte Fläche der Haltelasche der zweiten Buchse eingreift, um die erste Buchse an der zweiten Buchse zu befestigen.

5. Orthopädische Prothesenanordnung (10) nach Anspruch 4, wobei die Haltelasche (112) eine Mehrzahl von Haltelaschen enthält und der längliche Körper (104) eine Mehrzahl von Schlitzen (114) enthält, wobei jeder Schlitz der Mehrzahl von Schlitzen zwischen benachbarten Haltelaschen der Mehrzahl von Haltelaschen positioniert ist und jede Haltelasche der Mehrzahl von Haltelaschen zwischen benachbarten Schlitzen der Mehrzahl von Schlitzen positioniert ist.

6. Orthopädische Prothesenanordnung (10) nach Anspruch 5, wobei jede der Mehrzahl von Haltelaschen (112) der ersten Buchse (54) in einem der Mehrzahl von Schlitzen (114) der zweiten Buchse positioniert ist, um die erste Buchse an der zweiten Buchse zu befestigen.

7. Orthopädische Prothesenanordnung (10) nach Anspruch 6, wobei jede der Mehrzahl von Haltelaschen (112) der zweiten Buchse (54) in einem der Mehrzahl von Schlitzen (114) der ersten Buchse positioniert ist, um die erste Buchse an der zweiten Buchse zu befestigen.

## Revendications

1. Ensemble prothétique orthopédique (10) comprenant :
un composant fémoral (12) configuré pour être fixé à une extrémité distale du fémur d'un patient, le composant fémoral comprenant (i) une paire de condyles (22), (ii) une encoche intercondylienne (28) définie entre les condyles, et (iii) un alésage postérieur (30) s'étendant dans une direction médiale-latérale à travers la paire de condyles,
un plateau tibial (18) configuré pour être fixé à une extrémité proximale du tibia du patient, le plateau tibial comprenant une plateforme (32) et un montant s'étendant de manière distale à partir de la plateforme,
un insert tibial (16) comprenant (i) un corps (36) ayant une paire de surfaces (38, 40) configurées pour mettre en prise des surfaces de condyle (24, 26) correspondantes des condyles du composant fémoral, (ii) une tige (33) s'étendant de manière distale à partir du corps et dimensionnée pour être reçue dans une cavité définie dans la plateau tibial, (iii) une épine (44) s'étendant de manière proximale à partir du corps, et (iv) un trou de broche (42) s'étendant à travers l'épine de l'insert tibial, et une broche de charnière (56), dans lequel l'épine comprend une ouverture médiale (46), une ouverture latérale (48) et une paroi interne (50) s'étendant entre les ouvertures médiale et latérale afin de définir le trou de broche, **caractérisé par** :
une paire de douilles (54) dimensionnées et formées afin d'être positionnées dans le trou de broche de l'épine,
dans lequel la broche de charnière (56) est dimensionnée et formée afin d'être positionnée dans la paire de douilles et l'alésage postérieur afin de coupler l'insert tibial au composant fémoral, et
dans lequel chaque douille a une bride annulaire (102) et un corps allongé (104) s'étendant à partir de la bride annulaire, le corps allongé comprenant une languette de retenue (112), la languette de retenue d'une première douille étant dimensionnée et formée pour se fixer sur la languette de retenue de la seconde douille afin de fixer la paire de douilles sur l'épine.

2. Ensemble prothétique orthopédique (10) selon la revendication 1, dans lequel la languette de retenue (112) de chaque douille (54) comprend une paire de surfaces coudées (118) s'étendant à partir de la surface externe (116) du corps allongé (104) jusqu'à une surface d'extrémité (117).

3. Ensemble prothétique orthopédique (10) selon la revendication 2, dans lequel la languette de retenue (112) de chaque douille (54) comprend un axe longitudinal (110) et les surfaces coudées se coudent vers l'extérieur à partir de l'axe longitudinal du corps allongé (104) jusqu'à la surface d'extrémité (117).

4. Ensemble prothétique orthopédique (10) selon la revendication 3, dans lequel une surface coudée (118) de la languette de retenue (112) de la première douille (54) met en prise une surface coudée de la languette de retenue de la seconde douille afin de fixer la première douille sur la seconde douille.

5. Ensemble prothétique orthopédique (10) selon la revendication 4, dans lequel la languette de retenue (112) comprend une pluralité de languettes de retenue et le corps allongé (104) comprend une pluralité de fentes (114), dans lequel chaque fente de la pluralité de fentes est positionnée entre des languettes de retenue adjacentes de la pluralité de languettes de retenue et chaque languette de retenue de la pluralité de languettes de retenue est positionnée entre des fentes adjacentes de la pluralité de fentes.

6. Ensemble prothétique orthopédique (10) selon la revendication 5, dans lequel chacune de la pluralité de languettes de retenue (112) de la première douille (54) est positionnée à l'intérieur de l'une de la pluralité de fentes (114) de la seconde douille afin de fixer la première douille sur la seconde douille.

7. Ensemble prothétique orthopédique (10) selon la revendication 6, dans lequel chacune de la pluralité de languettes de retenue (112) de la seconde douille (54) est positionnée à l'intérieur de l'une de la pluralité de fentes (114) de la première douille afin de fixer la première douille sur la seconde douille.
